# EUROPÄISCHE PATENTSCHRIFT

(11) **EP 3 540 434 B1**
(45) Veröffentlichungstag und Bekanntmachung des Hinweises auf die Patenterteilung: **29.04.2020**
(21) Anmeldenummer: 18161732.5
(22) Anmeldetag: 14.03.2018
(51) Int. Cl.: G01N 33/24, G01N 21/85

(54) **TECHNIK ZUM MESSEN EINES MATRIXPOTENTIALS UND/ODER EINES DAMPFDRUCKS IN EINEM ZU UNTERSUCHENDEN MEDIUM**
TECHNIQUE FOR MEASURING A MATRIX POTENTIAL AND/OR A VAPOUR PRESSURE IN A MEDIUM TO BE INVESTIGATED
TECHNIQUE DE MESURE D'UN POTENTIEL MATRICIEL ET/OU D'UNE PRESSION DE LA VAPEUR DANS UN MILIEU À ÉTUDIER

(43) Veröffentlichungstag der Anmeldung: 18.09.2019
(73) Patentinhaber: Meter Group AG, 81379 München (DE)
(72) Erfinder: HIRTE, Josef, 80799 München (DE); VON UNOLD, Georg, 80638 München (DE)
(74) Vertreter: Lambacher, Michael

(56) Entgegenhaltungen:
- CN-U- 204 630 922
- DE-A1- 10 121 326
- JP-B2- 2 878 778
- JP-B2- 5 184 264
- US-A- 4 266 878
- US-A- 4 634 856
- LEE SEE GOH ET AL: "Hetero-core spliced optical fiber SPR sensor system for soil gravity water monitoring in agricultural environments", COMPUTERS AND ELECTRONICS IN AGRICULTURE, Bd. 101, 20. Januar 2014 (2014-01-20), Seiten 110-117, XP055500700, AMSTERDAM, NL ISSN: 0168-1699, DOI: 10.1016/j.compag.2013.12.008

## Beschreibung

### Gebiet der Technik

Die Erfindung betrifft eine Vorrichtung zum Messen eines Matrixpotentials und/oder eines Dampfdruckes in einem zu untersuchenden Medium. Die Erfindung betrifft ferner ein Chiplabor umfassend die erfindungsgemäße Messvorrichtung, sowie ein Verfahren zum Messen eines Matrixpotentials und oder eines Dampfdruckes in dem zu untersuchenden Medium unter Verwendung der Vorrichtung.

### Stand der Technik

Das Matrixpotential sowie auch der Dampfdruck sind wichtige Parameter zur Charakterisierung des Wasserhaushaltes in einem in sich geschlossenen System (z.B. Erdboden, aber auch z.B. ein Lebensmittel oder Arzneimittel).

Das Matrixpotential ist das Maß für die Summe der Halte- bzw. Matrixkräfte (umfassend Wasserstoffbrücken, Kapillarkräfte und van der Waals-Kräfte) für z.B. das Wasser im Boden. Es ist entscheidend für pflanzenphysiologische Untersuchungen, da Pflanzen dieses Potential überwinden müssen, um dem Boden Wasser zu entziehen. Das Matrixpotential ist jedoch ebenfalls eine entscheidende Größe bei der Beurteilung von Lebens- oder Arzneimitteln. Anhand des Matrixpotentials eines Lebens- oder Arzneimittels lassen sich z.B. Rückschlüsse auf deren Haltbarkeit, Textur oder geschmackliche Eigenschaften ziehen. Die Kenntnis über das Matrixpotential von Lebens- oder Arzneimitteln ist damit z.B. ein wichtiger Indikator für die Bestimmung der Verpackungsart, des Transport und der Lagerung der Lebens- oder Arzneimittel.

Zur Messung des Matrixpotentials in Böden werden beispielsweise Tensiometer eingesetzt. Sie bestehen in der Regel aus einem Hohlraum, der vollständig mit Wasser gefüllt ist. Dieser steht mit dem Boden über eine semipermeable Membran (vorzugsweise eine feinporige Keramik) in Verbindung, die wasserdurchlässig ist und Gase sperrt. Die Membran überträgt das Matrixpotential über die feinporige Struktur auf das Tensiometerwasser, wodurch ein messbarer atmosphärischer Unterdruck entsteht. Dieser kann mit Hilfe eines Drucksensors gemessen und zur Anzeige gebracht werden. Ferner sind bereits optische Tensiometer zur Messung des Matrixpotentials in Böden bekannt, wie beispielsweise aus der Patentschrift DE 10 047 937 C1.

Aus der US 4 634 856 A ist ferner eine Technik zum Messen einer Feuchtigkeitsänderung anhand optischer Lichtleitfasern bekannt, wobei an der Lichtleitfaser eine poröse Umhüllung angebracht ist, die ihre optischen Eigenschaften in Abhängigkeit von der Wasseraufnahme verändert.

In den Patentschriften JP 2 878778 B2 und JP 5 184264 B2 sind Methoden und Vorrichtungen zum Messen eines Flüssigkeitsstandes mittels optischer Messmethoden beschrieben. Ein Lichtleiter umfasst einen Spalt, der sich in Abhängigkeit eines Flüssigkeitslevels außerhalb des Lichtleiters durch Kapillarkräfte mit Flüssigkeit füllt. Abhängig vom Füllstand im Spalt ändert sich die Intensität des durch den Lichtleiter transmittierten Lichts, wodurch Rückschlüsse auf den Flüssigkeitsstand in der Umgebung des Lichtleiters gezogen werden können.

Neben dem Matrixpotential ist auch der Dampfdruck eine wichtige Größe zur Bestimmung des Wasserhaushalts in einem zu untersuchenden Medium. Durch das Wirken der Matrixkräfte auf das Wasser in dem zu untersuchenden Medium verändern sich Eigenschaften des Wassers, wie beispielsweise sein Dampfdruck, sein Gefrierpunkt und sein Siedepunkt. Eine Bestimmung des Dampfdruckes lässt im Umkehrschluss also auch Aussagen über die Matrixkräfte und den Wasserhaushalt des Mediums zu.

Zur Messung des Dampfdruckes in Lebensmitteln wird z.B. ein Verfahren angewandt, bei dem der Taupunkt der Luft in einem versiegelten Kopfraum oberhalb einer Lebensmittelprobe gemessen wird.

Die der vorliegenden Erfindung zugrunde liegende Aufgabe besteht darin, den Stand der Technik zu verbessern. Insbesondere soll es ermöglicht werden, die Messzeiten und Genauigkeit beim Messen eines Matrixpotentials und/oder Dampfdruckes in einem zu untersuchenden Medium unter Berücksichtigung technologischer und ökonomischer Aspekte zu verbessern.

### Kurzer Abriss der Erfindung

Zur Lösung der oben genannten sowie weiterer Aufgaben wird eine Vorrichtung zum Messen eines Matrixpotentials und/oder eines Dampfdruckes in einem zu untersuchenden Medium bereitgestellt. Die Vorrichtung umfasst eine Lichtwellenleiteinrichtung, in welcher ein schmaler Spalt ausgebildet ist, der dazu geeignet ist, einen Kapillareffekt bei Flüssigkeiten hervorzurufen, und dessen eines Ende mit dem zu untersuchenden Medium in Kontakt steht, wobei der Spalt einen vom Matrixpotential und/oder Dampfdruck in dem zu untersuchenden Medium abhängigen Wasserpegel aufweist. Ferner umfasst die Vorrichtung eine Lichtquelle, die dazu ausgelegt ist, Licht mit einer vorgebbaren Eingangsintensität in die Lichtwellenleiteinrichtung einzubringen, so dass das Licht durch den Spalt transmittiert wird, sowie eine Messeinrichtung, die dazu ausgelegt ist, eine Ausgangsintensität des Lichts an einer Stelle der Lichtwellenleiteinrichtung zu messen, an der das Licht den Spalt wenigstens einmal transmittiert hat. Die Vorrichtung umfasst ferner eine Recheneinrichtung, die dazu ausgelegt ist, auf der Grundlage der gemessenen Ausgangsintensität wenigstens einen Parameter zu ermitteln, der auf das Matrixpotential und/oder den Dampfdruck in dem zu untersuchenden Medium hinweist.

Der Spalt weist eine Eingangsöffnung und eine der Eingangsöffnung gegenüberliegende Ausgangsöffnung auf. Die Vorrichtung kann so ausgebildet sein, dass die Eingangsöffnung mit dem zu untersuchenden Medium in Verbindung steht. Ebenso kann die Ausgangsöffnung des Spalts mit dem zu untersuchenden Medium in Verbindung stehen. Die Ausgangsöffnung des Spalts kann auch mit der Umgebungsluft in Verbindung stehen. Ferner kann ein permeables oder semipermeables Medium als Übertragungsmedium eingesetzt werden, um eine Verbindung zwischen dem Spalt und dem zu untersuchenden Medium herzustellen.

Das zu untersuchende Medium kann ein beliebiges, ein Matrixpotential und/oder einen Dampfdruck aufweisendes Medium sein. Zum Beispiel kann das zu untersuchende Medium eine Erdbodenprobe sein. Auch kann das zu untersuchende Medium ein Lebensmittel, ein Arzneimittel oder die Umgebungsluft sein.

Die Lichtquelle kann eine LED (von engl. "light-emitting diode") sein. Die LED kann dazu ausgelegt sein, Licht in einem sichtbaren Spektrum auszusenden. Die Wellenlänge des von der Lichtquelle ausgestrahlten Lichts kann entsprechend im Bereich zwischen 380 nm und 780 nm liegen. Vorzugsweise kann die Wellenlänge des von der Lichtquelle ausgestrahlten Lichts im Spektralbereich zwischen 400 nm und 500 nm (blaues Licht) liegen. Die Messeinrichtung kann eine Photodiode sein.

Die vorgebbare Eingangsintensität des Lichtes kann durch einen Benutzer einstellbar sein. Alternativ kann die vorgebbare Eingangsintensität des Lichtes ein fest vorgegebener Wert sein.

Der wenigstens eine Parameter, welcher auf das Matrixpotential und/oder den Dampfdruck in dem zu untersuchenden Medium hinweist, kann beispielsweise einen Druck, Feuchte (in %) oder Wasseraktivität (in %) umfassen.

Die Lichtwellenleiteinrichtung der Vorrichtung umfasst ein erstes Bauteil und ein zweites Bauteil, wobei das erste Bauteil und das zweite Bauteil jeweils eine Grenzfläche aufweisen, welche die Kapillarwände des Spalts (d.h. die Spaltwände) bilden.

Das erste Bauteil der Lichtwellenleiteinrichtung kann ein erster Lichtwellenleiter sein. Dieser erste Lichtwellenleiter kann an einer von seiner Grenzfläche verschiedenen Endfläche mit der Lichtquelle in Verbindung stehen. Der Lichtwellenleiter ist dazu ausgebildet, das in den Lichtwellenleiter eingekoppelte Licht (annähernd) verlustfrei zu leiten. Der erste Lichtwellenleiter kann so ausgebildet sein, dass er nur an seiner Grenzfläche und an der Endfläche, an der er mit der Lichtquelle in Verbindung steht, lichtdurchlässig ist. Der erste Lichtwellenleiter kann beispielsweise eine längliche Form mit einem runden oder eckigen Querschnitt aufweisen. Gemäß einer Variante kann die Endfläche des ersten Lichtwellenleiters der Grenzfläche des ersten Lichtwellenleiters gegenüber liegen.

Die Grenzfläche des ersten Lichtwellenleiters kann eine ebene Fläche sein. Die Grenzfläche des ersten Lichtwellenleiters kann auch eine Wölbung aufweisen oder anderweitig geformt sein. Die Grenzfläche des ersten Lichtwellenleiters kann als polierte oder als unpolierte Fläche ausgebildet sein. Die Gestaltung der Grenzfläche des ersten Lichtwellenleiters richtet sich nach der gewünschten Form des Spalts.

Gemäß einer Variante kann das zweite Bauteil der Lichtwellenleiteinrichtung ein zweiter Lichtwellenleiter sein, welcher an einer von seiner Grenzfläche verschiedenen Endfläche mit der Messeinrichtung in Verbindung steht. Der zweite Lichtwellenleiter kann derart ausgeführt sein, dass er nur an seiner Grenzfläche und an der Endfläche, an der er mit der Messeinrichtung in Verbindung steht, lichtdurchlässig ist. Der zweite Lichtwellenleiter kann beispielsweise eine längliche Form mit einem runden oder eckigen Querschnitt aufweisen. Gemäß einer Variante kann die Endfläche des zweiten Lichtwellenleiters der Grenzfläche des zweiten Lichtwellenleiters gegenüber liegen. In diesem Falle können die Grenzfläche und die Endfläche des Lichtwellenleiters senkrecht zu einer Längsachse des ersten Lichtwellenleiters angeordnet sein.

In einem Grundzustand ist der zwischen dem ersten Bauteil und dem zweiten Bauteil gebildete Spalt "leer", das heißt, er ist vollständig mit Umgebungsluft oder einem anderen ausgewählten Gas gefüllt. Der erste und der zweite Lichtwellenleiter weisen einen von dem Gas in dem leeren Spalt verschiedenen Brechungsindex auf. Durch den Unterschied der Brechungsindizes der Lichtwellenleiter und des leeren Spalts wird das Licht, das von der Lichtquelle in den ersten Lichtwellenleiter eingestrahlt wird, an der Grenzfläche des ersten Lichtwellenleiters gebrochen. Dadurch wird der Einfallswinkel des durch den Spalt transmittierten Lichtes auf die Grenzfläche zum zweiten Lichtwellenleiter verringert. Unterschreitet der Einfallswinkel des Lichtes auf die Grenzfläche des zweiten Lichtwellenleiters einen kritischen Wert, so wird das Licht an dieser Grenzfläche total reflektiert und koppelt nicht in den zweiten Lichtwellenleiter ein.

Wenn der Spalt mit dem zu untersuchenden Medium in Verbindung steht, füllt er sich in Abhängigkeit von dem auf den Spalt übertragenen Matrixpotential und/oder Dampfdruck des zu untersuchenden Mediums mit Wasser. Dabei gilt, je höher das Matrixpotential bzw. der Dampfdruck in dem zu untersuchenden Medium, desto höher steigt der Wasserpegel in dem Spalt. In einem Spaltabschnitt, in dem der Spalt mit Wasser gefüllt ist, ändert sich der Brechungsindex in dem Spalt von beispielsweise 1 (für Luft) auf 1,33 (für Wasser). Dadurch verändert sich die Differenz zwischen dem Brechungsindex des jeweiligen Lichtwellenleiters und dem Brechungsindex des Spalts (d.h. dem Brechungsindex des Mediums mit dem der Spalt in dem betreffenden Abschnitt gefüllt ist). Mit anderen Worten ändern sich die Brechungseigenschaften an der Grenzfläche des ersten bzw. des zweiten Lichtwellenleiters, was zu einer Änderung der gemessenen Ausgangsintensität des Lichtes bei einem mit Wasser gefüllten Spalt im Vergleich zu einem leeren Spalt führt.

Alternativ zu einem zweiten Lichtwellenleiter kann das zweite Bauteil der Lichtwellenleiteinrichtung ein Spiegel sein. Entsprechend kann die Grenzfläche des zweiten Bauteils die Spiegelfläche des Spiegels sein. Die Spiegelfläche ist dazu ausgelegt, Licht, das durch den Spalt transmittiert, in Richtung der Grenzfläche des ersten Bauteils (des ersten Lichtwellenleiters) zurück zu reflektieren. Gemäß dieser Ausführungsform können die Lichtquelle und die Messeinrichtung an einer gemeinsamen von der Grenzfläche verschiedenen Endfläche des ersten Bauteils angeordnet sein.

Unabhängig davon, ob das zweite Bauteil als Lichtwellenleiter oder als Spiegel ausgebildet ist, kann die Grenzfläche des zweiten Bauteils eine ebene, gewölbte, oder anderweitig geformte Fläche sein. Die Gestaltung der Grenzfläche des zweiten Lichtwellenleiters richtet sich nach der gewünschten Form des Spalts.

Die Grenzfläche des ersten Bauteils und die Grenzfläche des zweiten Bauteils sind in einem spitzen Winkel zueinander angeordnet. Entsprechend weist der Spalt einen sich verjüngenden Querschnitt auf. Dabei kann der Spalt an dem verjüngten Ende mit dem zu untersuchenden Medium in Verbindung stehen. Ein V-förmiger Spalt kann gewisse Vorteile gegenüber einem parallelwandigen Spalt aufweisen. Beispielsweise kann der Messbereich durch die V-Form vergrößert werden, wobei im Falle geringen Matrixpotentials bzw. geringen Dampfdruckes, durch den geringen Spaltquerschnitt im Bereich der Spaltmündung eine hohe Messgenauigkeit erreicht werden kann. Eine Wölbung der Grenzfläche des zweiten Bauteils kann zusätzlich eine Linearisierung der Messwerte bezwecken.

Die Vorrichtung zum Messen eines Matrixpotentials und/oder eines Dampfdruckes in einem zu untersuchenden Medium kann eine erste semipermeable Membran umfassen. Die erste semipermeable Membran kann zwischen dem Spalt und dem zu untersuchenden Medium derart angeordnet sein, dass der Spalt mit dem zu untersuchenden Medium in Verbindung steht. Mit anderen Worten kann die erste semipermeable Membran das Matrixpotential und/oder den Dampfdruck in dem zu untersuchenden Medium auf den Spalt übertragen. Dabei kann die erste semipermeable Membran dazu ausgelegt sein, den Spalt nach außen gegen das Eindringen unerwünschter Substanzen oder Schmutzpartikel in den Spalt abzuschirmen. Die erste semipermeable Membran kann eine feinporige Keramik sein, die wasser- und luftdurchlässig ist. Durch die erste semipermeable Membran kann es ermöglicht werden, dass keine unerwünschten Schmutzpartikel oder ähnliches in den Spalt geraten und diesen verstopfen.

Ferner kann die Vorrichtung eine zweite semipermeable Membran umfassen, die aus einem elastischen Material aufgebaut ist und zwischen der ersten semipermeablen Membran und dem Spalt angeordnet ist, so dass der Spalt mit dem zu untersuchenden Medium in Verbindung steht. Die zweite semipermeable Membran kann dazu ausgelegt sein, einen Kontakt zwischen der ersten semipermeablen Membran und dem Spalt herzustellen. Die zweite semipermeable Membran kann aus einem feinporigen elastischen Material aufgebaut sein, das wasser- und luftdurchlässig ist. Die zweite semipermeable Membran kann vorteilhaft sein, wenn das Matrixpotential des zu untersuchenden Mediums gemessen werden soll, da dazu der Spalt in direktem Kontakt zu dem Porenwasser des zu untersuchenden Mediums stehen muss.

Alternativ kann im Falle einer Dampfdruckmessung in dem zu untersuchenden Medium zwischen der ersten semipermeablen Membran und dem Spalt ein Luftspalt ausgebildet sein. Für die Messung des Dampfdruckes ist es nämlich entscheidend, dass der Spalt mit der Luft in dem zu untersuchenden Medium in Verbindung steht.

Zur Lösung der der Erfindung zugrunde liegenden Aufgabe wird ferner ein Chiplabor (oder auch lab-on-a-chip, kurz: "LOC") zum Messen eines Matrixpotentials und/oder eines Dampfdruckes in einem zu untersuchenden Medium bereitgestellt. Das Chiplabor umfasst ein Trägerelement und eine Vorrichtung gemäß einer der oben beschriebenen Varianten, wobei die Vorrichtung auf dem Trägerelement des Chiplabors ausgebildet ist. Ferner umfasst das Chiplabor eine Steuereinrichtung, die mit der Lichtquelle, der Messeinrichtung und/oder der Recheneinrichtung der Vorrichtung in Verbindung steht und die dazu ausgelegt ist, wenigstens eine der Lichtquelle, der Messeinrichtung und der Recheneinrichtung zu steuern.

Das Trägerelement kann eine Leiterplatte sein. Vorzugsweise kann das Chiplabor zwei Vorrichtungen gemäß einer der oben beschriebenen Varianten umfassen. Vorzugsweise können die beiden Vorrichtungen den gleichen Aufbau aufweisen. Beide Vorrichtungen können mit Licht der gleichen Eingangsintensität betrieben werden. Die eine der zwei Vorrichtungen kann von dem zu untersuchenden Medium isoliert sein und Referenzmesswerte für eine Offsetmessung liefern. Somit können Störeinflüsse, die die Ausgangsintensität des Lichtes neben der Reflexion an dem Spalt verändern, durch einen Vergleich mit der Referenz-Vorrichtung eliminiert werden.

Das Chiplabor kann ferner wenigstens eine Schnittstelle umfassen. Die wenigstens eine Schnittstelle kann mit einer externen Recheneinrichtung zur Weiterverarbeitung und/oder Ausgabe des durch die Recheneinrichtung ermittelten wenigstens einen Parameters verbunden werden. Ferner kann das Chiplabor über die wenigstens eine Schnittstelle mit einer Eingabeeinrichtung verbunden werden, die dazu ausgelegt ist, Befehle an die Steuereinrichtung zur Steuerung der wenigstens einen Vorrichtung auf dem Chiplabor zu senden.

Die wenigstens eine Vorrichtung oder Teile davon (wie beispielsweise die Lichtwelleinleiteinrichtung) können auf dem Chiplabor durch ein Lithografie-Verfahren realisiert werden.

Zur Lösung der der Erfindung zugrunde liegenden Aufgabe wird ferner ein Verfahren zum Messen eines Matrixpotentials und/oder eines Dampfdruckes in einem zu untersuchenden Medium mittels einer erfindungsgemäßen Vorrichtung bereitgestellt. Das Verfahren umfasst das Bereitstellen der Vorrichtung in dem zu untersuchenden Medium, so dass der Spalt mit dem zu untersuchenden Medium in Verbindung tritt. Weiter umfasst das Verfahren das Einstrahlen von Licht mit einer vorgebbaren Eingangsintensität in die Lichtwellenleiteinrichtung der Vorrichtung, so dass das Licht den in der Lichtwellenleiteinrichtung ausgebildeten Spalt transmittiert. Außerdem umfasst das Verfahren das Messen einer Ausgangsintensität des Lichtes durch die Messeinrichtung der Vorrichtung, nachdem es den Spalt wenigstens einmal transmittiert hat, sowie das Ermitteln, mit Hilfe der Recheneinrichtung, wenigstens eines Parameters, der auf das Matrixpotential und/oder den Dampfdruck in dem zu untersuchenden Medium hinweist, auf der Grundlage der gemessenen Ausgangsintensität des Lichtes.

Das Einstrahlen des Lichts kann gepulst erfolgen. Mit anderen Worten kann in (vorgebbaren) regelmäßigen Zeitabständen ein Licht-Puls in das erste Bauteil (den ersten Lichtwellenleiter) der Lichtwellenleiteinrichtung eingebracht werden. Die Messung der Ausgangsintensität kann mit Pulsfolge des eingestrahlten Lichts synchronisiert werden.

Der Verfahrensschritt des Ermitteins kann ferner Folgendes umfassen: Vergleichen der Ausgangsintensität mit der vorgebbaren Eingangsintensität des Lichtes; Bestimmen einer Intensitätsänderung des Lichts durch Vergleich der Eingangsintensität mit der Ausgangsintensität; und Berechnen, anhand der Intensitätsänderung, des wenigstens einen auf des Matrixpotential und/oder den Dampfdruck hinweisenden Parameters.

### Kurze Beschreibung der Figuren

Anhand von Zeichnungen sollen im Folgenden Aspekte der vorliegenden Erfindung beispielhaft verdeutlicht werden. Es zeigen:
Figuren 1a und 1b - Varianten einer erfindungsgemäßen Vorrichtung zum Messen eines Matrixpotentials und/oder eines Dampfdruckes in einem zu untersuchenden Medium;
Figuren. 2a und 2b - schematisch das Prinzip der Lichtbrechung an den Kapillarwänden (Grenzflächen der Lichtwellenleiter) gemäß der in Figur 1a dargestellten Vorrichtung;
Figuren 3a und 3b - weitere Varianten einer erfindungsgemäßen Vorrichtung;
Figuren 4a und 4b - schematisch das Prinzip der Lichtbrechung an den Kapillarwänden (Grenzflächen der Lichtwellenleiter) gemäß einer der in den Figuren 3a und 3b dargestellten Vorrichtungsvarianten;
Figur 5 - ein Beispiel für eine Ausgestaltung der Kapillarwände in der Lichtwellenleiteinrichtung einer erfindungsgemäßen Vorrichtung;
Figur 6 - eine Variante eines erfindungsgemäßen Chiplabors; und
Figur 7 - ein erfindungsgemäßes Verfahren zum Messen eines Matrixpotentials und/oder eines Dampfdruckes in einem zu untersuchenden Medium.

### Detaillierte Figurenbeschreibung

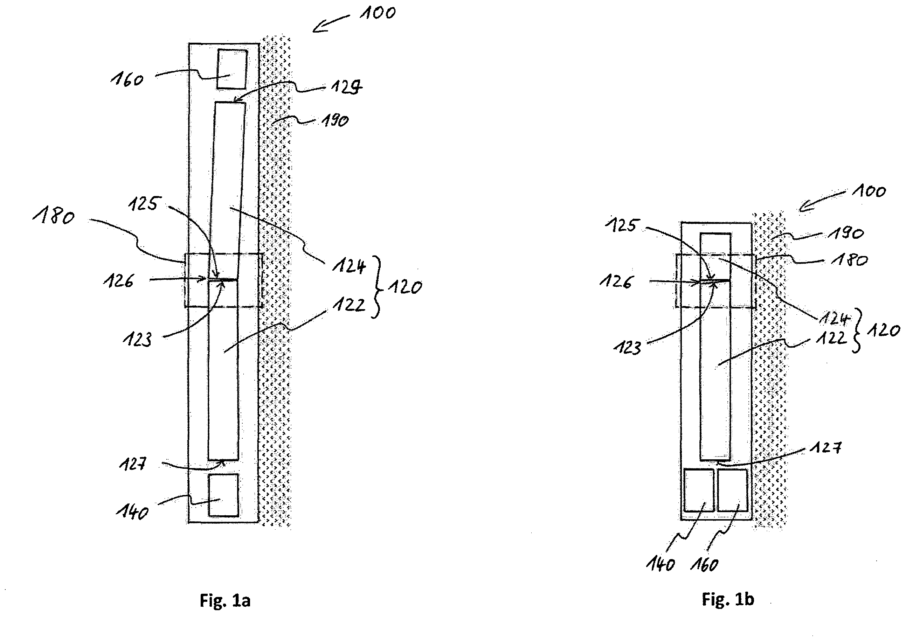

Im Folgenden werden die in den Figuren 1a und 1b dargestellten Ausführungsbeispiele näher erläutert. Die Figuren 1a und 1b zeigen jeweils eine alternative Variante einer erfindungsgemäßen Vorrichtung 100 zum Messen eines Matrixpotentials und/oder eines Dampfdruckes in einem zu untersuchenden Medium 190. Jede der in den Figuren 1a und 1b dargestellten Vorrichtungen 100 umfasst eine Lichtwellenleiteinrichtung 120. Ferner umfasst die Vorrichtung 100 eine Lichtquelle 140 und eine Messeinrichtung 160.

Die Vorrichtung 100 umfasst ferner eine Recheneinrichtung (nicht dargestellt), die dazu ausgelegt ist, wenigstens einen Parameter zu ermitteln, der auf das Matrixpotential und/oder den Dampfdruck hinweist.

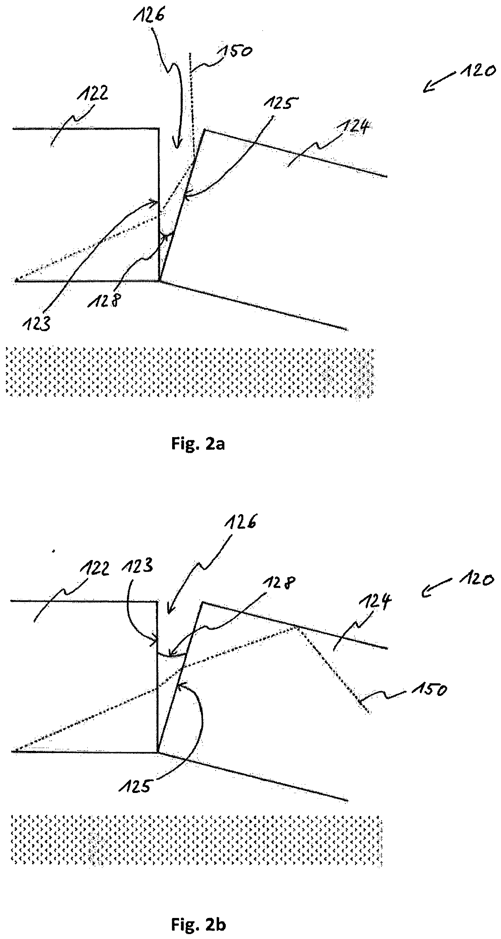

Die Lichtwellenleiteinrichtung 120 umfasst ein erstes Bauteil 122 und ein zweites Bauteil 124. Zwischen dem ersten Bauteil 122 und dem zweiten Bauteil 124 ist ein schmaler Spalt 126 ausgebildet, der einen V-förmigen Querschnitt aufweist, wie aus der vergrößerten Darstellung in den Figuren 2a und 2b genauer hervorgeht. Das erste Bauteil 122 weist an seiner dem zweiten Bauteil 124 zugewandten Seite eine Grenzfläche 123 auf. Das zweite Bauteil 124 weist seinerseits an seiner dem ersten Bauteil 122 zugewandten Seite eine Grenzfläche 125 auf, so dass die Grenzfläche 123 des ersten Bauteils 122 und die Grenzfläche 125 des zweiten Bauteils 124 die Kapillarwände des Spalts 126 bilden.

Gemäß den Figuren 1a und 1b umfasst die Vorrichtung 100 ferner eine semipermeable Membran 180. Der Spalt 126 steht über die semipermeable Membran 180 mit dem zu untersuchenden Medium 190 in Verbindung. Alternativ kann der Spalt 126 auch direkt mit dem zu untersuchenden Medium 190 in Verbindung stehen. Die semipermeable Membran 180 wirkt jedoch als Filtereinheit und beugt einer Verschmutzung oder Verstopfung des Spalts 126 vor. Die semipermeable Membran 180 kann vorzugsweise aus einem feinporigen Material (z.B. einer feinporigen Keramik) aufgebaut sein. Sie kann ferner wasser- sowie luftdurchlässig sein. Die semipermeable Membran 180 überträgt das in dem zu untersuchenden Medium 190 vorhandene Porenwasser und/oder den in dem zu untersuchenden Medium 190 herrschenden Dampfdruck auf das verjüngte Ende des Spalts 126. Durch Kapillareffekte füllt sich der Spalt 126. Der Wasserpegel in dem Spalt 126 hängt hierbei von dem Matrixpotential und/oder von dem Dampfdruck in dem zu untersuchenden Medium 190 ab. Je höher dabei das Matrixpotential bzw. der Dampfdruck an dem verjüngten Ende des Spalts 126 ist, desto höher steigt das Wasser im Spalt 126.

Gemäß Figur 1a ist das erste Bauteil 122 ein erster Lichtwellenleiter 122 und das zweite Bauteil 124 ein zweiter Lichtwellenleiter 124. Gemeinsam bilden der erste Lichtwellenleiter 122 und der zweite Lichtwellenleiter 124 einen Strahlengang, der von dem Spalt 126 unterbrochen ist. An der der Grenzfläche 123 entgegengesetzten freien Endfläche 127 des ersten Lichtwellenleiters 122 ist die Lichtquelle 140 angeordnet. Am anderen Ende des Strahlengangs, nämlich an der der Grenzfläche 125 entgegengesetzten freien Endfläche 129 des zweiten Lichtwellenleiters 124, ist die Messeinrichtung 160 angeordnet.

Wird Licht von der Lichtquelle 140 in den ersten Lichtwellenleiter 122 eingestrahlt, so wird ein Teil der Lichtstrahlen an der Grenzfläche 123 des ersten Lichtwellenleiters 122 reflektiert und ein Teil in den Spalt 126 transmittiert. Das durch den Spalt 126 transmittierte Licht wird wiederum an der Grenzfläche 125 des zweiten Lichtwellenleiters 124 reflektiert, oder tritt in den zweiten Lichtwellenleiter 124 ein. Wieviel Licht an den Grenzflächen 123, 125 des ersten bzw. des zweiten Lichtwellenleiters reflektiert wird, hängt von der Brechungsindexdifferenz zwischen dem jeweiligen Lichtwellenleiter 122, 124 und dem im Spalt 126 befindlichen Medium ab. Wenn das auf den Spalt 126 übertragene Matrixpotential und/oder der Dampdruck gering ist, ist der Wasserpegel im Spalt 126 entsprechend niedrig. Der Spalt 126 ist somit überwiegend mit Luft gefüllt, die einen deutlich geringeren Brechungsindex als Wasser (1<1,33) aufweist, was bewirkt, dass verhältnismäßig viel des durch den Spalt 126 transmittierten Lichtes an der Grenzfläche zum zweiten Lichtwellenleiter 124 reflektiert wird. Mit anderen Worten, je niedriger der Wasserstand in dem Spalt 126 ist, desto mehr Licht wird an der Grenzfläche des zweiten Lichtwellenleiters 124 aus dem Strahlengang ausgekoppelt. Das heißt auch, dass bei niedrigem Wasserpegel in dem Spalt 126 verhältnismäßig wenig Lichtwellen in den zweiten Lichtwellenleiter 124 einkoppeln und die Messeinrichtung 160 am Ende des Strahlengangs erreichen. Je höher dagen der Wasserpegel im Spalt 126 ist, desto weniger Licht wird an den Grenzflächen 123, 125 reflektiert und desto mehr Licht tritt in den zweiten Lichtwellenleiter 124 ein. Je höher also das Matrixpotential und/oder der Dampfdruck in dem mit dem Spalt 126 in Kontakt stehenden Medium, desto größer ist die am Ende des Strahlengangs gemessene Ausgangsintensität im Verhältnis zur Eingangsintensität des in den ersten Lichtwellenleiter eingestrahlten Lichtes.

Die Vorrichtung gemäß Figur 1b unterscheidet sich von der Vorrichtung gemäß Figur 1a darin, dass das zweite Bauteil 124 ein Spiegel 124 und die Grenzfläche 125 des zweiten Bauteils 124 die Spiegelfläche 125 des Spiegels 124 ist. Das auf die Spiegelfläche 125 treffende Licht wird gemäß dieser Variante vollständig reflektiert und durch den Spalt 126 zurück in Richtung des ersten Lichtwellenleiters 122 gelenkt. Wiederum abhängig vom Wasserpegel im Spalt 126 wird ein kleinerer oder größerer Teil des eingestrahlten Lichts an den Wänden des Spalts 126 (Grenzflächen 123, 125) reflektiert. Ein verbleibender Teil der Lichtstrahlen wird dagegen zurück in den ersten Lichtwellenleiter 122 geleitet. Die Messeinrichtung 160 zum Messen einer Ausgangsintentsität des Lichtes am Ende des Strahlengangs ist gemäß der Vorrichtung 100 in Figur 1b an der gleichen Endfläche 127 des ersten Lichtwellenleiters 122 angeordnet, an der auch die Lichtquelle 140 angeordnet ist.

In den Figuren 2a und 2b ist der Vorgang der Lichtbrechung an der Grenzfläche 123 des ersten Bauteils 122 und an der Grenzfläche 125 des zweiten Bauteils 124 noch einmal schematisch dargestellt. Figur 2a zeigt eine Lichtwellenleiteinrichtung 120 gemäß der Variante in Figur 1a, wobei der Spalt 126 einen geringen Wasserpegel 128 aufweist. Beim Übergang von dem ersten Lichtwellenleiter 122 in den Spalt 126 (d.h. an den Spaltwänden bzw. den Grenzflächen 123, 125 der Lichtwellenleiter 122, 125) wird ein Lichtstrahl 150 gebrochen. Wird ein kritischer Winkel unterschritten, in dem der Lichtstrahl 150 auf eine der Grenzflächen 123 bzw. 125 der Lichtwellenleiter 122 bzw. 124 trifft, so tritt Totalreflexion auf und der Lichtstrahl wird an der entsprechenden Grenzfläche 123 bzw. 125 vollständig reflektiert. Ist der Spalt 126, wie in Figur 2a dargestellt, überwiegend mit Luft gefüllt, so wird ein Teil der in den Spalt 126 eintretenden Lichtstrahlen wie dargestellt abgelenkt und an der Grenzfläche 125 des zweiten Lichtwellenleiters 124 (total) reflektiert. Die auf diese Weise reflektierten Lichtstrahlen erreichen die Messeinheit 160 nicht, weshalb die gemessene Ausgangsintensität gegenüber der Eingangsintensität des Lichtes abnimmt.

Figur 2b zeigt den Vorgang der Lichtbrechung analog zu Figur 2a und mit dem gleichen Einfallwinkel des Lichtstrahls 150 auf die Grenzfläche 123 des ersten Lichtwellenleiters 122. Der Unterschied zu der Darstellung in Figur 2a besteht darin, dass der Spalt 126 einen vergleichsweise hohen Wasserpegel 128 aufweist. Da Wasser einen deutlich höheren Brechungsindex als Luft aufweist (1,33 gegenüber 1), verändert sich die Brechungsindexdifferenz zwischen dem Spalt 126 und dem entsprechenden Lichtwellenleiter und damit die Brechungseigenschaften an den Grenzflächen 123, 125 der Lichtwellenleiter 122, 124 derart, dass der Lichtstrahl 150 einen kritischen Winkel (bzgl. der Grenzfläche 125) für die Totalreflexion an der Grenzfläche 125 des zweiten Lichtwellenleiters 124 nicht unterschreitet und in den zweiten Lichtwellenleiter 124 einkoppelt. Auf diese Weise geht weniger Licht an dem Spalt 126 durch Totalreflexion verloren und die gemessene Ausgangsintensität am Ende des Strahlengangs ist höher im Vergleich zu der in Figur 2a dargestellten Konstellation.

Durch den Öffnungswinkel und die Geometrie der Spaltwände kann unter anderem der Messbereich der Vorrichtung 100 festgelegt werden. Dabei gilt, je größer der Winkel der Grenzflächen 123 und 125 zueinander, desto größer ist auch der Messbereich, da das Fassungsvolumen des Spalts 126 sich vergrößert.

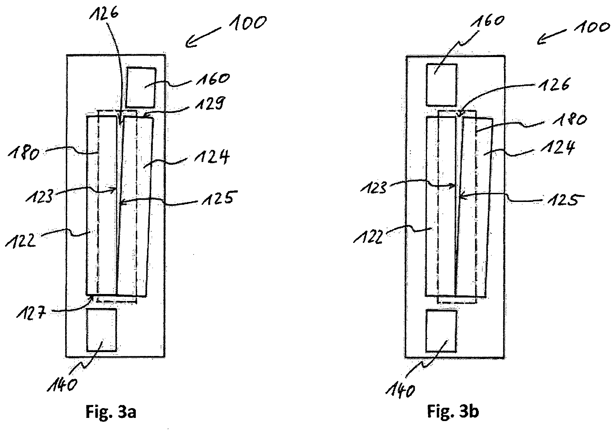

Im Folgenden werden weitere Varianten einer erfindungsgemäßen Vorrichtung gemäß den Figuren 3a und 3b näher erläutert. Die in den Figuren 3a und 3b dargestellten Varianten einer erfindungsgemäßen Vorrichtung 100 umfassen die gleichen Komponenten wie die in Figur 1a dargestellte Vorrichtung. Entsprechend sind in den Figuren 3a und 3b die gleichen Bezugszeichen wie in Figur 1a verwendet. Im Gegensatz zu der in Figur 1a dargestellten Variante,.sind jedoch die Lichtwellenleiter 122 und 124 der Vorrichtungen gemäß den Figuren 3a und 3b nicht hintereinander (in Reihe) angeordnet, sondern nebeneinander (parallel). Das grundlegende Messprinzip ist jedoch das gleiche. Lediglich die Grenzflächen 123, 125 des ersten Lichtwellenleiters 122 und des zweiten Lichtwellenleiters 124 befinden sich nun an den einander zugewandten Längsseiten der beiden Lichtwellenleiter 122, 124. Der von den Grenzflächen 123, 125 der Lichtwellenleiter 122, 124 gebildete Spalt 126 kann somit länger ausgebildet sein und ein größeres Fassungsvermögen für Wasser aufweisen als eine Vorrichtung 100 gemäß Figur 1a.

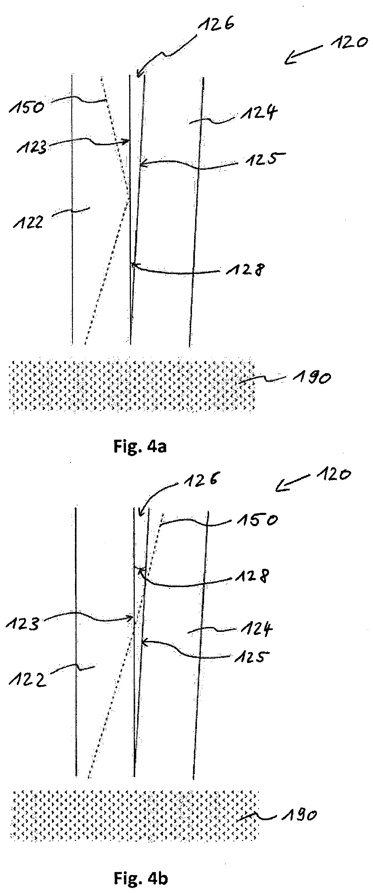

In den Figuren 4a und 4b ist, analog zu den Figuren 2a und 2b, das Prinzip der Lichtbrechung in Abhängigkeit vom Wasserpegel 128 in dem Spalt 126 für die Vorrichtungsvarianten gemäß den Figuren 3a und 3b dargestellt. Abhängig von dem Wasserpegel 128 in dem Spalt 126 wird ein größerer oder ein kleinerer Teil des in den ersten Lichtwellenleiter 122 eingestrahlten Lichts an einer der Grenzflächen 123, 125 der Lichtwellenleiter 122, 124 (total) reflektiert. Durch Messung der Intensität des in den zweiten Lichtwellenleiter 124 geleiteten Lichts gemäß der Variante in Figur 3a oder aber durch Messung des reflektierten Lichts gemäß Figur 3b, kann so auf das Matrixpotential und/oder auf den Dampfdruck des Mediums geschlossen werden, mit dem der Spalt 126 in Verbindung steht.

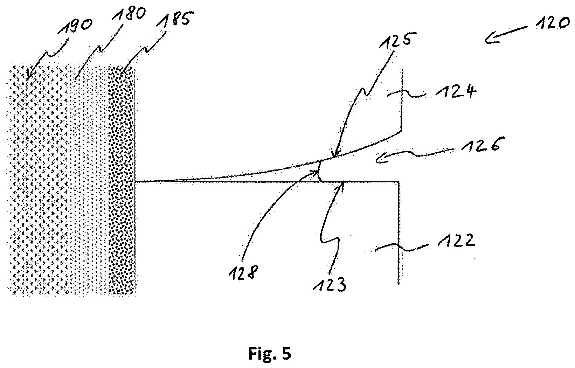

In Zusammenhang mit der Figur 5 wird im Folgenden eine geometrische Ausgestaltung des Spalts 126 beispielhaft erläutert. Figur 5 zeigt einen ersten Lichtwellenleiter 122 und einen zweiten Lichtwellenleiter 124, wobei die Grenzflächen 123, 125 der Lichtwellenleiter 122, 124 einen sich verjüngenden Spalt 126 bilden. Die Grenzfläche 123 des ersten Lichtwellenleiters 122 ist als ebene, polierte Fläche ausgebildet. Die Grenzfläche 125 des zweiten Lichtwellenleiters 124 weist dagegen im Querschnitt eine leichte Wölbung auf. Gegenüber einem Spalt 126 mit einfachem V-förmigem Querschnitt, der von zwei jeweils ebenen Grenzflächen 123, 125 gebildet wird, kann ein Spalt 126 mit einer gewölbten Grenzfläche 125 vorteilhaft sein. Durch eine entsprechend geformte Wölbung der Grenzfläche 125 können bestimmte Messbereiche linearisiert werden, da die Spaltbreite eines sich füllenden Spalts 126 logarithmisch von der zu messenden Wasserspannung oder dem zu messenden Dampfdruck abhängt. Folgt die Verjüngung des Spalts 126 nicht einer linearen, sondern einer logarithmischen Funktion, besteht letztendlich ein linearer Zusammenhang zwischen der Wasserspannung bzw. dem Dampfdruck und der Füllhöhe des Spalts 126. Ein weiterer Vorteil der sich gewölbt aufweitenden Querschnittsform des Spalts 126 besteht darin, dass im Bereich kleiner Matrixpotentiale bzw. geringen Dampfdruckes sehr genaue Messergebnisse erzielt werden können, da die Grenzflächen 123, 125 am verjüngten Ende des Spalts 126 in einem sehr spitzen Winkel zueinander verlaufen und einen sehr filigranen Spaltquerschnitt bilden. Die geschwungene Aufweitung des Spalts 126 hin zu seinem offenen Ende vergrößert gleichzeitig das maximale Füllvolumen des Spalts 126 und somit den Messbereich.

Wie in Figur 5 dargestellt, ist das verjüngte Ende des Spalts 126 dem zu untersuchenden Medium 190 zugewandt. Zwischen dem zu untersuchenden Medium 190 und dem Spalt 126 sind ferner eine erste semipermeable Membran 180 und eine zweite semipermeable Membran 185 angeordnet. Beide Membrane 180, 185 weisen eine feinporige Strukur auf und sind wasser- und luftdurchlässig. Die Membrane 180, 185 dienen dem Transport des Porenwassers bzw. der Luft in dem zu untersuchenden Medium 190 hin zur Mündung des Spalts 126. Während die erste semipermeable Membran 180 in erster Linie eine Filterfunktion hat und verhindert, dass Schmutzpartikel oder ähnliches in den Spalt 126 gelangen, ist die zweite semipermeable Membran 185 aus einem elastischen Material aufgebaut und schmiegt sich an die Lichtwellenleiter 122, 124 an, um eine zuverlässige und stetige Verbindung der ersten semipermeablen Membran 180 mit dem Spalt 126 zu gewährleisten. Im Falle einer Messung von Dampfdruck in einem zu untersuchenden Medium 190 kann anstatt der zweiten semipermeablen Membran 185 ein Luftspalt zwischen der ersten semipermeablen Membran 180 und dem Spalt 126 angeordnet sein. Auch kann in einem solchen Fall gänzlich auf eine Membran zwischen dem zu untersuchenden Medium 190 und dem Spalt 126 verzichtet werden. Zur Messung des Dampfdruckes ist es entscheidend, dass der Spalt 126 mit der Luft in dem zu untersuchenden Medium 190 in Verbindung steht. Da kein direkter Kontakt hergestellt werden muss, ist auch eine abschirmende Filtereinrichtung nicht notwendig.

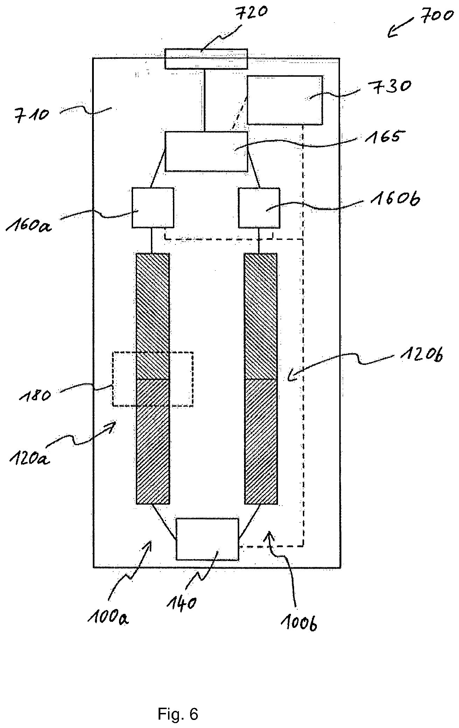

Im Folgenden wird ein Chiplabor gemäß der Darstellung in Figur 6 näher erläutert. Das Chiplabor 700 umfasst eine Trägerplatte 710, sowie eine Vorrichtung 100a gemäß der in Figur 1a dargestellten Variante. Das Chiplabor 700 umfasst außerdem eine Vorrichtung 100b, die analog zu der Vorrichtung 100a aufgebaut ist mit dem Unterschied, dass die Vorrichtung 100b von dem zu untersuchenden Medium isoliert ist. Eine Lichtquelle 140 strahlt Licht mit der jeweils gleichen Intensität in die Lichtwellenleiteinrichtung 120a, 120b beider Vorrichtungen 100a und 100b ein. Es versteht sich, dass die Eingangsintensiät des Lichtes durch einen Benutzer einstellbar sein kann. Am Ende jeder Lichtwellenleiteinrichtung 120a und 120b ist jeweils eine Messeinrichtung 160a, 160b angeordnet, die die Ausgangsintensität des Lichtes am Ende des entsprechenden Strahlengangs erfasst. Die Messeinrichtungen 160a und 160b senden die gemessenen Intensitätswerte an eine Recheneinrichtung 165, welche die jeweils gemessenen Ausgangsintensitäten vergleicht und anhand des Vergleichs das Matrixpotential und/oder den Dampfdruck berechnet, welches/r von dem zu untersuchenden Medium auf den Spalt 126 der Vorrichtung 100a übertragen wurde. Das Chiplabor 700 weist ferner eine Schnittstelle 720 auf, welche dazu ausgelegt ist, die von der Recheneinrichtung 165 bereitgestellten Daten an eine externe Datenverarbeitungseinrichtung weiterzuleiten. Ferner weist das Chiplabor eine Steuereinrichtung 730 auf, die mit der Lichtquelle 140, der Messeinrichtung 160 und/oder der Recheneinrichtung 165 in Verbindung steht. Mit Hilfe der Steuereinrichtung kann die Lichtquelle 140, die Messeinrichtung 160 und/oder die Recheneinrichtung 165 angesteuert werden.

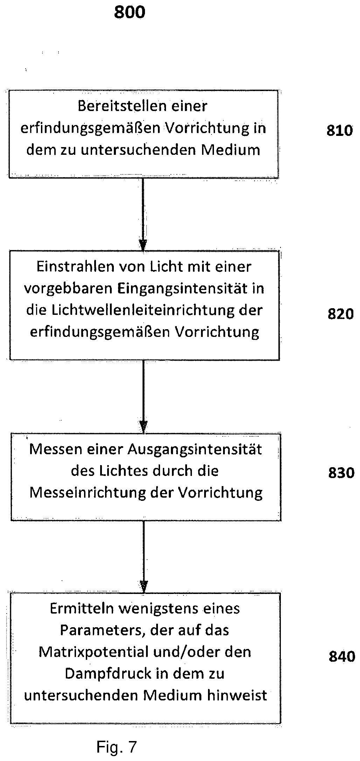

Im Folgenden wird ein erfindungsgemäßes Verfahren zum Messen eines Matrixpotentials und/oder eines Dampfdruckes in einem zu untersuchenden Medium gemäß Figur 7 näher erläutert.

In einem ersten Verfahrensschritt 810 wird wenigstens eine erfindungsgemäße Vorrichtung 100 in dem zu untersuchenden Medium 190 bereitgestellt. Dabei ist es von nebengeordneter Bedeutung, ob das zu untersuchende Medium 190 die Vorrichtung 100 vollständig umschließt. Entscheidend ist lediglich, dass eine Verbindung zwischen dem zu untersuchenden Medium 190 und dem Spalt 126 der Vorrichtung 100 hergestellt wird.

In einem zweiten Verfahrensschritt 820 wird Licht mit einer vorgebbaren Eingangsintensität in die Lichtwellenleiteinrichtung 120 der Vorrichtung 100 eingestrahlt. Das Licht wird durch den ersten Lichtwellenleiter 122 zum Spalt 126 geleitet, um anschließend über das zweite Bauteil 124 (zweiter Lichtwellenleiter 124 oder Spiegel 124) der Lichtwellenleiteinrichtung 120 zur Messeinrichtung 160 der Vorrichtung 100 zu gelangen. Es versteht sich, dass die vorgebbare Intensität des Lichtes beliebig gewählt werden kann. Es ist jedoch wichtig, dass die Eingangsintensität des Lichtes bekannt ist, damit sie für die Bestimmung eines Intensitätsverlustes als Referenz herangezogen werden kann.

Ein dritter Verfahrensschritt 830 umfasst das Messen einer Ausgangsintensität des in Schritt 720 eingestrahlten Lichtes. Die Ausgangsintensität des Lichtes ist diejenige Intensität des Lichtes, die von der Messeinrichtung 160 erfasst wird. Ein Vergleich der Ausgangsintensität und der Eingangsintensität lässt auf einen Intensitätsverlust zwischen der Ausgangsintensität und der Eingangsintensität des Lichtes schließen.

In einem vierten Verfahrensschritt 840 wird wenigstens ein Parameter ermittelt, der auf das Matrixpotential und/oder den Dampfdruck in dem zu untersuchenden Medium 190 hinweist. Das Matrixpotential und/oder der Dampfdruck in dem zu untersuchenden Medium 190 verhalten sich proportional zu dem Wasserpegel 128 in dem Spalt 126, die mit dem zu untersuchenden Medium 190 in Kontakt steht. Mit ansteigendem Wasserpegel 128 in dem Spalt 126 wiederum wird weniger Licht an den Spaltwänden (Grenzflächen 123, 125) reflektiert und der Intensitätsverlust des Lichtes wird reduziert.

Der Verfahrensschritt 840 des Ermittelns des Matrixpotentials und/oder des Dampfdruckes in dem zu untersuchenden Medium 190 kann ein Vergleichen der Ausgangsintensität mit der vorgebbaren Eingangsintensität des Lichtes umfassen. Ferner kann das Ermitteln des Matrixpotentials und/oder des Dampfdruckes das Bestimmen einer Änderung der Intensität des Lichts zwischen der Eingangsintensität und der Ausgangsintensität und ein anschließendes Berechnen des Matrixpotentials und/oder des Dampfdruckes in dem zu untersuchenden Medium anhand der Intensitätsänderung umfassen.

Zur Kalibrierung der Vorrichtung 100 und zur Eliminierung von Einflüssen, die die Messergebnisse verfälschen können, können Messergebnisse einer identischen, parallel betriebenen Vorrichtung, welche von dem zu untersuchenden Medium isoliert ist, als Vergleichswerte herangezogen werden.

Es versteht sich, dass das oben beschriebene Verfahren ebenfalls mit einem erfindungsgemäßen Chiplabor durchgeführt werden kann.

Durch die in dieser Offenbarung beschriebenen technischen Mittel kann eine Verbesserung herkömmlicher Messtechniken zum Messen eines Matrixpotentials und/oder eines Dampfdruckes in einem zu untersuchenden Medium erzielt werden.

Insbesondere kann bei der Gestaltung der die Kavität bildenden Grenzflächen durch entsprechende Gestaltung der Grenzflächenoberflächen bzw. Funktionalisierung der Oberflächen (z.B. Bereitstellung von hydrophilen Grenzflächen) der Messbereich der beschriebenen Technik hochsensitiv ausgeprägt werden. Ein weiterer Vorteil gegenüber herkömmlichen Matrixpotentialsensoren besteht darin, dass die vorliegende Technik eine weitere Miniaturisierung zulässt, da die Lichtwellenleiter sehr klein ausgeführt werden können (z.B. bei Realisierung durch Lithographie). Dadurch ist die vorliegende Messtechnik für minimalinvasive Anwendungen geeignet. Ein weiterer Vorteil der vorliegenden Technik besteht darin, dass im Gegensatz zu Druckaufnehmertensiometern die Kavität nicht befüllt werden muss und somit nahezu wartungsfrei ist. Ferner ist die Ansprechgeschwindigkeit im Vergleich zu üblichen Matrixpotentialsensoren sehr gut. Die beschriebene optische Messtechnik hat gegenüber herkömmlichen Verfahren auch den Vorteil, dass sie viel energiesparender arbeitet. Somit ist die vorliegende Technik für batteriegepufferte Remote-IOT-Anwendungen hervorragend geeignet. Wird ferner eine Referenzkavität verwendet, wie oben beschrieben, kann eine Offsetdriftkompensation sowie ein Plausibilitäts-Selbsttest realisiert werden. Somit sind vertrauenswürdige und rauscharme Messungen möglich.

## Patentansprüche

1. Vorrichtung (100) zum Messen eines Matrixpotentials und/oder eines Dampfdruckes in einem zu untersuchenden Medium (190), umfassend:
eine Lichtwellenleiteinrichtung (120), in welcher ein schmaler Spalt (126) ausgebildet ist, der dazu geeignet ist, einen Kapillareffekt bei Flüssigkeiten hervorzurufen, und der mit dem zu untersuchenden Medium (190) in Kontakt gebracht werden kann, wobei der Spalt (126) einen vom Matrixpotential und/oder Dampfdruck in dem zu untersuchenden Medium (190) abhängigen Wasserpegel (128) aufweist, und wobei die Lichtwellenleiteinrichtung (120) ein erstes Bauteil (122) und ein zweites Bauteil (124) umfasst, wobei das erste Bauteil (122) und das zweite Bauteil (124) jeweils eine Grenzfläche (123, 125) aufweisen, welche die Kapillarwände des Spalts (126) bilden;
eine Lichtquelle (140), die dazu ausgelegt ist, Licht mit einer vorgebbaren Eingangsintensität in die Lichtwellenleiteinrichtung (120) einzubringen, so dass das Licht durch den Spalt (126) transmittiert wird;
eine Messeinrichtung (160), die dazu ausgelegt ist, eine Ausgangsintensität des Lichts an einer Stelle der Lichtwellenleiteinrichtung (120) zu messen, an der das Licht den Spalt (126) wenigstens einmal transmittiert hat; und
eine Recheneinrichtung (165), die dazu ausgelegt ist, auf der Grundlage der gemessenen Ausgangsintensität wenigstens einen Parameter zu ermitteln, der auf das Matrixpotential und/oder den Dampfdruck in dem zu untersuchenden Medium (190) hinweist; **dadurch gekennzeichnet, dass** die Grenzfläche (123) des ersten Bauteils (122) und die Grenzfläche (125) des zweiten Bauteils (124) in einem spitzen Winkel zueinander angeordnet sind und somit der Spalt (126) einen sich verjüngenden Querschnitt aufweist.

2. Vorrichtung (100) nach Anspruch 1, wobei das zweite Bauteil (124) ein Spiegel (124) ist, und wobei die Lichtquelle (140) und die Messeinrichtung (160) an einer gemeinsamen von der Grenzfläche verschiedenen Endfläche (127) des ersten Bauteils (122) angeordnet sind.

3. Vorrichtung (100) nach Anspruch 1 oder 2, wobei die Grenzfläche (125) des zweiten Bauteils (124) eine ebene oder gewölbte Fläche ist.

4. Vorrichtung (100) nach einem der vorhergehenden Ansprüche, wobei das erste Bauteil (122) ein erster Lichtwellenleiter (122) ist, welcher an einer von seiner Grenzfläche (123) verschiedenen Endfläche (127) mit der Lichtquelle (140) in Verbindung steht.

5. Vorrichtung (100) nach Anspruch 3, wobei die Grenzfläche (123) des ersten Lichtwellenleiters (122) eine ebene Fläche ist.

6. Vorrichtung (100) nach einem der vorhergehenden Ansprüche, wobei das zweite Bauteil (124) ein zweiter Lichtwellenleiter (124) ist, welcher an einer von seiner Grenzfläche (125) verschiedenen Endfläche (129) mit der Messeinrichtung (160) in Verbindung steht.

7. Vorrichtung (100) nach einem der vorhergehenden Ansprüche, ferner umfassend: eine erste semipermeable Membran (180), die derart am Spalt (126) angeordnet ist, dass der Spalt (126) mit dem zu untersuchenden Medium (190) in Verbindung steht.

8. Vorrichtung (100) nach Anspruch 7, ferner umfassend:
eine zweite semipermeable Membran (185), die aus einem elastischen Material aufgebaut ist und zwischen der ersten semipermeablen Membran (180) und dem Spalt (126) angeordnet ist, so dass der Spalt (126) mit dem zu untersuchenden Medium (190) in Verbindung steht.

9. Vorrichtung (100) nach Anspruch 7, wobei zum Messen des Dampfdruckes ein Luftspalt zwischen der ersten semipermeablen Membran (180) und dem Spalt (126) ausgebildet ist.

10. Chiplabor (700) zum Messen eines Matrixpotentials und/oder eines Dampfdruckes in einem zu untersuchenden Medium (190), umfassend:
ein Trägerelement (710);
eine Vorrichtung (100) gemäß einem der vorhergehenden Ansprüche, wobei die Vorrichtung (100) auf dem Trägerelement (710) des Chiplabors (700) ausgebildet ist; und
eine Steuereinrichtung (730), die mit der Lichtquelle (140), der Messeinrichtung (160) und/oder der Recheneinrichtung (165) der Vorrichtung (100) in Verbindung steht, und die dazu ausgelegt ist, die Lichtquelle (140), die Messeinrichtung (160) und/oder die Recheneinrichtung (165) zu steuern.

11. Verfahren (800) zum Messen eines Matrixpotentials und/oder eines Dampfdruckes in einem zu untersuchenden Medium (190) mittels einer Vorrichtung (100) gemäß einem der Ansprüche 1 bis 9, wobei das Verfahren (800) die folgenden Verfahrensschritte umfasst:
Bereitstellen (810) der Vorrichtung (100) in dem zu untersuchenden Medium (190), so dass der Spalt (126) der Vorrichtung (100) mit dem zu untersuchenden Medium (190) in Verbindung tritt;
Einstrahlen (820) von Licht mit einer vorgebbaren Eingangsintensität in die Lichtwellenleiteinrichtung (120) der Vorrichtung (100), so dass das Licht den in der Lichtwellenleiteinrichtung (120) ausgebildeten Spalt (126) transmittiert;
Messen (830) einer Ausgangsintensität des Lichtes durch die Messeinrichtung (160) der Vorrichtung (100), nachdem es den Spalt (126) wenigstens einmal transmittiert hat; und
Ermitteln (840), mit Hilfe der Recheneinrichtung (165), wenigstens eines Parameters, der auf das Matrixpotential und/oder den Dampfdruck in dem zu untersuchenden Medium (190) hinweist, auf der Grundlage der gemessenen Ausgangsintensität des Lichtes.

12. Verfahren (800) nach Anspruch 11, wobei der Verfahrensschritt des Ermittelns (840) umfasst:
Vergleichen der Ausgangsintensität mit der vorgebbaren Eingangsintensität des Lichtes;
Bestimmen einer Intensitätsänderung des Lichts durch Vergleich der Eingangsintensität mit der Ausgangsintensität; und
Berechnen, anhand der Intensitätsänderung, des wenigstens einen auf das Matrixpotential und/oder den Dampfdruck hinweisenden Parameters.

## Claims

1. A device (100) for measuring a matrix potential and/or a vapour pressure in a medium to be examined (190), comprising:
an optical waveguide apparatus (120), in which a narrow gap (126) is formed which is suitable for causing a capillary effect in liquids and which is adapted to be brought into contact with the medium to be examined (190), wherein the gap (126) has a water level (128) which is dependent on the matrix potential and/or vapour pressure in the medium to be examined (190), and wherein the optical waveguide apparatus (120) comprises a first component (122) and a second component (124), wherein the first component (122) and the second component (124) each have a boundary surface (123, 125), which forms the capillary walls of the gap (126);
a light source (140) designed to introduce light with a predetermined input intensity into the optical waveguide apparatus (120) such that the light is transmitted through the gap (126);
a measuring apparatus (160) designed to measure an output intensity of the light at a location of the optical waveguide apparatus (120) at which the light has transmitted the gap (126) at least once; and
a computing apparatus (165) designed to determine, on the basis of the measured output intensity, at least one parameter indicative of the matrix potential and/or the vapour pressure in the medium to be examined (190);
**characterised in that** the boundary surface (123) of the first component (122) and the boundary surface (125) of the second component (124) are arranged at an acute angle to one another and therefore the gap (126) has a tapering cross-section.

2. The device (100) according to claim 1, wherein the second component (124) is a mirror (124), and wherein the light source (140) and the measuring apparatus (160) are arranged at a common end surface (127) of the first component (122) which is different from the boundary surface.

3. The device (100) according to claim 1 or 2, wherein the boundary surface (125) of the second component (124) is a flat or curved surface.

4. The device (100) according to any of the preceding claims, wherein the first component (122) is a first optical waveguide (122) which is in connection with the light source (140) at an end surface (127) different from its boundary surface (123).

5. The device (100) according to claim 3, wherein the boundary surface (123) of the first optical waveguide (122) is a flat surface.

6. The device (100) according to any of the preceding claims, wherein the second component (124) is a second optical waveguide (124) which is connected to the measuring apparatus (160) at an end surface (129) different from its boundary surface (125).

7. The device (100) according to any of the preceding claims, further comprising:
a first semipermeable membrane (180), which is arranged at the gap (126) such that the gap (126) is in connection with the medium to be examined (190).

8. The device (100) according to claim 7, further comprising:
a second semipermeable membrane (185) made of an elastic material and arranged between the first semipermeable membrane (180) and the gap (126) such that the gap (126) is in connection with the medium to be examined (190).

9. The device (100) according to claim 7, wherein an air gap is formed between the first semipermeable membrane (180) and the gap (126) for measuring the vapour pressure.

10. A chip lab (700) for measuring a matrix potential and/or a vapour pressure in a medium to be examined (190), comprising:
a support member (710);
a device (100) according to any of the preceding claims, wherein the device (100) is formed on the support member (710) of the chip lab (700); and
a control apparatus (730) which is connected to the light source (140), the measuring apparatus (160) and/or the computing apparatus (165) of the device (100) and which is designed to control the light source (140), the measuring apparatus (160) and/or the computing apparatus (165).

11. A method (800) for measuring a matrix potential and/or a vapour pressure in a medium to be examined (190) by means of a device (100) according to any of the claims 1 to 9, wherein the method (800) comprises the following method steps:
providing (810) the device (100) in the medium to be examined (190) such that the gap (126) of the device (100) is in connection with the medium to be examined (190);
directing (820) light with a predetermined input intensity into the optical waveguide apparatus (120) of the device (100) such that the light transmits the gap (126) formed in the optical waveguide apparatus (120);
measuring (830) an output intensity of the light by the measuring apparatus (160) of the device (100) after it has transmitted the gap (126) at least once; and
determining (840), with the aid of the computing apparatus (165), at least one parameter indicative of the matrix potential and/or the vapour pressure in the medium to be examined (190), on the basis of the measured output intensity of the light.

12. The method (800) according to claim 11, wherein the method step of determining (840) comprises:
comparing the output intensity with the predetermined input intensity of the light;
determining a change in intensity of the light by comparing the input intensity with the output intensity; and
calculating, from the change in intensity, at least one parameter indicative of the matrix potential and/or the vapour pressure.

## Revendications

1. Dispositif (100) de mesure d'un potentiel matriciel et/ou d'une pression de la vapeur dans un milieu (190) à étudier, comprenant :
un dispositif de guide d'onde optique (120), dans lequel une fente étroite (126) est formée, laquelle est appropriée pour provoquer un effet capillaire chez les liquides, et qui peut être mise en contact avec le milieu à étudier (190), dans lequel la fente (126) a un niveau d'eau (128) qui dépend du potentiel matriciel et/ou de la pression de vapeur dans le milieu (190) à étudier, et dans lequel le dispositif de guide d'onde optique (120) comprend un premier composant (122) et un second composant (124), dans lequel le premier composant (122) et le second composant (124) ont chacun une interface (123, 125) qui forme les parois capillaires de la fente (126) ;
une source de lumière (140), qui est conçue pour introduire de la lumière avec une intensité d'entrée prédéterminable dans le dispositif de guide d'onde optique (120), de sorte que la lumière est transmise à travers la fente (126) ;
un dispositif de mesure (160) qui est conçu pour mesurer une intensité de sortie de la lumière à un emplacement du dispositif de guide d'onde optique (120) auquel la lumière a transmis la fente (126) au moins une fois ; et
un dispositif informatique (165) qui est conçu pour déterminer sur la base de l'intensité de sortie mesurée au moins un paramètre qui indique le potentiel matriciel et/ou la pression de la vapeur dans le milieu (190) à étudier;
**caractérisé en ce que** l'interface (123) du premier composant (122) et l'interface (125) du second composant (124) sont disposées à un angle aigu l'une par rapport à l'autre et ainsi la fente (126) a une section transversale effilée.

2. Dispositif (100) selon la revendication 1, dans lequel le second composant (124) est un miroir (124), et dans lequel la source de lumière (140) et le dispositif de mesure (160) sont disposés sur une surface d'extrémité commune (127) du premier composant (122) différente de l'interface.

3. Dispositif (100) selon les revendications 1 ou 2, dans lequel l'interface (125) du second composant (124) est une surface plane ou incurvée.

4. Dispositif (100) selon l'une quelconque des revendications précédentes, dans lequel le premier composant (122) est un premier guide d'onde optique (122) qui est connecté à la source de lumière (140) au niveau d'une surface d'extrémité (127) qui est différente de son interface (123).

5. Dispositif (100) selon la revendication 3, dans lequel l'interface (123) du premier guide d'onde optique (122) est une surface plane.

6. Dispositif (100) selon l'une quelconque des revendications précédentes, dans lequel le second composant (124) est un second guide d'onde optique (124) qui est connecté au dispositif de mesure (160) au niveau d'une surface d'extrémité (129) qui est différente de son interface (125).

7. Dispositif (100) selon l'une quelconque des revendications précédentes, comprenant en outre :
une première membrane semi-perméable (180) qui est disposée sur la fente (126) de telle sorte que la fente (126) communique avec le milieu à étudier (190).

8. Dispositif (100) selon la revendication 7, comprenant en outre :
une seconde membrane semi-perméable (185), qui est constituée d'un matériau élastique et est disposée entre la première membrane semi-perméable (180) et la fente (126), de telle sorte que la fente (126) communique avec le milieu à étudier (190).

9. Dispositif (100) selon la revendication 7, dans lequel une lame d'air entre la première membrane semi-perméable (180) et la fente (126) est formée pour mesurer la pression de la vapeur.

10. Laboratoire sur puce (700) pour la mesure d'un potentiel matriciel et/ou d'une pression de la vapeur dans un milieu (190) à étudier, comprenant :
un élément de support (710) ;
un dispositif (100) selon l'une quelconque des revendications précédentes, dans lequel le dispositif (100) est formé sur l'élément de support (710) du laboratoire sur puce (700) ; et
un dispositif de commande (730), qui est connecté à la source lumineuse (140), au dispositif de mesure (160) et/ou au dispositif informatique (165) du dispositif (100), et qui est conçu pour commander la source lumineuse (140), le dispositif de mesure (160) et/ou le dispositif informatique (165).

11. Procédé (800) de mesure d'un potentiel matriciel et/ou d'une pression de la vapeur dans un milieu (190) à étudier à l'aide d'un dispositif (100) selon l'une quelconque des revendications 1 à 9, dans lequel le procédé (800) comprend les étapes de procédé suivantes :
installer (810) le dispositif (100) dans le milieu (190) à étudier, de sorte que la fente (126) du dispositif (100) communique avec le milieu à étudier (190) ;
irradier (820) de lumière à une intensité d'entrée prédéterminable le dispositif de guide d'onde optique (120) du dispositif (100), de sorte que la lumière transmet la fente (126) formée dans le dispositif de guide d'onde optique (120) ;
mesurer (830) une intensité de sortie de la lumière par le dispositif de mesure (160) du dispositif (100) après qu'elle a-transmis au moins une fois la fente (126) ; et
déterminer (840), à l'aide du dispositif informatique (165), au moins un paramètre qui indique le potentiel matriciel et/ou la pression de la vapeur dans le milieu (190) à étudier, sur la base de l'intensité de sortie mesurée de la lumière.

12. Procédé (800) selon la revendication 11, dans lequel l'étape de procédé de détermination (840) comprend :
la comparaison de l'intensité de sortie avec l'intensité d'entrée prédéterminable de la lumière ;
la détermination d'une variation de l'intensité de la lumière en comparant l'intensité d'entrée et l'intensité de sortie ; et
le calcul, en fonction de la variation de l'intensité, de l'au moins un paramètre indiquant le potentiel matriciel et/ou la pression de la vapeur.
